# EUROPÄISCHE PATENTSCHRIFT

(11) **EP 0 643 596 B1**
(45) Veröffentlichungstag und Bekanntmachung des Hinweises auf die Patenterteilung: **08.09.1999**
(21) Anmeldenummer: 94911091.0
(22) Anmeldetag: 30.03.1994
(51) Int. Cl.: A61M 16/00, A61M 16/08

(54) **EINRICHTUNG ZUR ÜBERWACHUNG WENIGSTENS EINER VERBINDUNG EINES MEDIZINISCHEN SCHLAUCHLEITUNGSSYSTEMS**
DEVICE FOR MONITORING AT LEAST ONE CONNECTION IN A MEDICAL TUBING SYSTEM
DISPOSITIF PERMETTANT LA SURVEILLANCE D'AU MOINS UN RACCORDEMENT DANS UN SYSTEME DE TUYAUX MEDICAL

(30) Priorität: 02.04.1993 DE 4310855
(43) Veröffentlichungstag der Anmeldung: 22.03.1995
(73) Patentinhaber: KLIMM, Josef, D-93051 Regensburg (DE); SCHMID, Thomas, D-93098 Mintraching (DE)
(72) Erfinder: KLIMM, Josef, D-93051 Regensburg (DE); SCHMID, Thomas, D-93098 Mintraching (DE)
(74) Vertreter: Graf, Helmut, Dipl.-Ing.
(86) Internationale Anmeldenummer: DE9400356
(87) Internationale Veröffentlichungsnummer: WO9422516

(56) Entgegenhaltungen:
- EP-A- 0 314 306
- FR-A- 2 298 147
- US-A- 3 595 228
- US-A- 3 942 526

## Beschreibung

Die Erfindung bezieht sich auf ein medizinisches Beatmungssystem, bestehend aus wenigstens zwei Elementen, von denen eines ein Y-Stück und eines ein mit dem Y-Stück verbundener Tubus ist.

Derartige Beatmungssysteme sind grundsätzlich bekannt und werden beispielsweise bei Operationen zusammen mit einer Narkosemaschine verwendet. Die Verbindung zwischen dem Y-Stück und dem Tubus ist so ausgebildet, daß sie durch Zusammenstecken schnell herstellbar ist. Nachteilig hierbei ist aber, daß sich die Verbindung zwischen dem Tubus und dem Y-Stück auch ungewollt leicht lösen kann, was zu schwerwiegenden Unfällen mit Todesfolge führen kann, und zwar insbesondere bei einem völlig beatmeten Patienten und insbesondere auch dann, wenn eine aktive Atmung, beispielsweise durch Verabreichung von Muskelrelaxanzien nicht möglich ist. Hinzu kommt, daß die Elemente des Beatmungssystems oftmals nur einmal verwendete Artikel sind, die preiswert und daher auch mit relativ großen Toleranzen bezüglich Querschnittsabmessungen usw. gefertigt werden, so daß allein schon wegen dieser Toleranzen die Gefahr von Diskonnektionen der Verbindungen nicht ausgeschlossen werden kann.

Bekannt ist eine Einrichtung zur Überwachung der Verbindung zwischen einem Anschlußstück und einem Tubus eines medizinischen Beatmungssystems (US-3 595 228). Zur Erzeugung eines Alarmsignal ist bei dieser bekannte Alarmeinrichtung ein Oszillator vorgesehen, der über eine Steuerleitung mit dem am Tubus angeschlossenen Bearbeitungssystem verbunden ist und über den Tubus mit der vom Patienten gebildeten elektrischen Masse. In diesem geerdeten Zustand wird der Oszillator des Alarmgerätes am Schwingen gehindert und damit auch zur Abgabe des Warnsignals. Tritt eine Diskonnektion auf, so soll bei dieser bekannten Alarmeinrichtung der Steuereingang des Oszillators freigegeben werden, so daß der Oszillator dann das akustische Warnsignal abgeben kann. Das bekannte System hat Nachteile. Insbesondere ist es nicht betriebssicher, da der Oszillator ebenso am Schwingen und damit zur Abgabe des Warnsignals dann gehindert ist, wenn zwar eine Diskonnektion eingetreten ist, das am Tubus sich anschließende Rohrstück aber beispielsweise auf der Haut des Patienten aufliegt. Auch in diesem Falle wäre trotz der aufgetretenen Diskonnektion der Steuereingang des Oszillators geerdet, so daß kein Alarmsignal abgegeben wird.

Bekannt ist weiterhin ein Alarmsystem US-A-3 942 526, mit welchem das Vorhanden einer Infusionsflüssigkeit in einem Infusionssystem überwacht wird. Auch hier geht es nicht um die Überwachung der Verbindung zwischen einem Y-Stück und einem Tubus eines medizinischen Bearbeitungssystems.

Bekannt ist ferner ein Sicherheitssystem für medizinische Flüssigkeitspumpen (EP-A- O 314 306). Es geht bei diesem bekannten Sicherheitssystem darum, bei einer Diskonnektion eines an die Pumpe angeschlossenen Schlauches die Pumpe abzustellen und dann auch einen Alarm auszulösen. Es handelt sich nicht um ein Beatmungssystem. Insbesondere geht es bei diesem bekannten Sicherheitssystem auch nicht darum, die Verbindung zwischen einem Y-Stück und dem Tubus eines Beatmungssystems zu überwachen.

Aufgabe der Erfindung ist es, ein Beatmungssystem aufzuzeigen, bei welchem die Verbindung zwischen dem Y-Stück und dem Tubus dieses Bearbeitungssystems zuverlässig überwacht wird.

Zur Lösung dieser Aufgabe ist ein Beatmungssystem entsprechend dem Patentanspruch 1 ausgebildet.

Bei dem erfindungsgemäßen Bearbeitungssystem ist zuverlässig eine Überwachung der Verbindung zwischen dem Tubus und dem Y-Stück möglich. Die erfindungsgemäße Ausbildung gewährleistet bei einfacher und robuster Konstruktion eine hohe Betriebssicherheit.

Das erste Sensorelement kann bei der Erfindung fest an dem dieses Sensorelement aufweisenden Element des Beatmungssystems vorgesehen oder einstückig mit diesem Element hergestellt sein. Der Permanentmagnet kann beispielsweise dadurch gebildet sein, daß in das Material des ersten Sensorelementes, beispielsweise Kunststoffmaterial als Füller ein magnetisiertes ferritisches Material eingebunden ist.

Weiterbildungen der Erfindung sind Gegenstand der Unteransprüche.

Die Erfindung wird im Folgenden anhand der Figuren an einem Ausführungsbeispiel näher erläutert. Es zeigen:

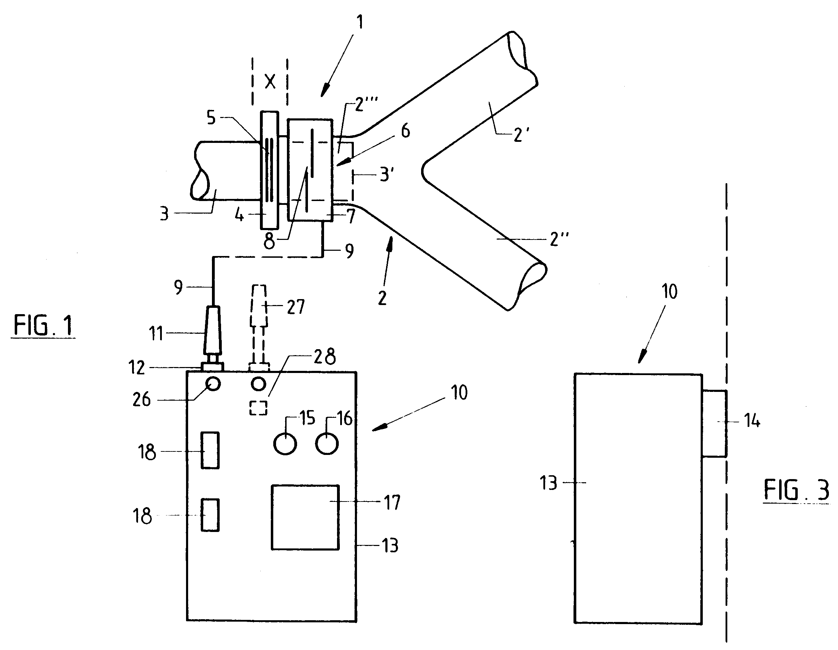

Fig. 1 in vergrößerter Detaildarstellung ein Y-Stück und ein mit diesem verbundener Tubus eines medizinischen Bearbeitungssystems, zusammen mit den am Tubus sowie am Y-Stück vorgesehenen Sensorelelementen und einer Überwachungs- und Anzeigeeinrichtung der Überwachungseinrichtung;
Fig. 2 ein Blockdiagramm der Überwachungs- und Anzeigeeinrichtung;
Fig. 3 in vereinfachter Darstellung und in Seitenansicht das Gehäuse der Überwachungs- und Anzeigeelektronik.

In den Figuren ist mit 1 die Verbindung zweier Komponenten oder Elementen eines medizinischen Schlauch-Leitungssystems, nämlich eines Beatmungssystems dargestellt, und zwar speziell zwischen einem Y-Stück 2 und einem Tubus 3. Das Y-Stück bildet in bekannter Weise einen Inspirations-Anschluß 2' und einen Expirations-Anschluß 2'', mit denen dieses Y-Stück an eine nicht dargestellte Narkose-Maschine oder an weitere, nicht dargestellte Elemente eines Beatmungssystems angeschlossen ist. Am Anschluß 2''' ist das Y-Stück 2 mit dem Tubus 3 verbunden, d.h. letzterer ist mit seinem einen Ende am Anschluß 2''' in das Y-Stück 2 eingeschoben und dort durch Klemmsitz gehalten. An diesem Ende weist der Tubus 3 bei der dargestellten Ausführungsform einen Flansch 4 auf, der in gleicher Weise wie der Tubus 3 aus Kunststoff gefertigt ist, und zwar vorzugsweise einstückig mit dem Tubus 3. In den Flansch 4 ist ein Permanentmagnet 5 eingebettet. Bei mit dem Y-Stück 2 ordnungsgemäß verbundenem Tubus 3 liegt der Flansch 4 einem am Y-Stück 2 befestigten Sensorelement 6 benachbart. Letzteres besteht aus einem vorzugsweise aus Kunststoff gefertigtem Gehäuse 7, welches unter Verwendung einer Klemm-Befestigung an dem den Anschluß 2''' bildenden Ende des Y-Stückes 2 außen lösbar befestigt ist, und zwar beispielsweise mit Hilfe eines Clip, vorzugsweise mit Hilfe eines am Gehäuse 7 angeformten Clip. Die Befestigungsmittel für das Sensorelement 6 und/oder das Y-Stück sind so ausgebildet, daß eine Befestigung des Sensorelementes 6 am Y-Stück 2 nur in einer eine einwandfreie Funktion des Überwachungssystems gewährleistenden Lage möglich ist. Im Inneren des Gehäuses 7 weist das Sensorelement 6 einen Reed-Kontakt 8 auf. Dieser ist so ausgebildet, daß er im Ruhezustand bzw. im nicht aktivierten Zustand geöffnet ist.

Ist die Verbindung 1 ordnungsgemäß hergestellt und befindet dementsprechend der Flansch 4 mit dem Permanentmagneten 5 dem Sensorelement 6 unmittelbar benachbart, so ist der Reed-Kontakt 8 geschlossen. Über ein Verbindungskabel 9 ist der Reed-Kontakt 8 mit einer Überwachungs- und Anzeigeeinrichtung 10 verbunden, und zwar mittels eines an der Verbindungsleitung 9 vorgesehenen Mehrfach-Steckers 11, der in eine Mehrfach-Buchse 12 am Gehäuse 13 der Überwachungs- und Anzeigeelektronik einsteckbar ist.

Die Überwachungs- und Anzeigeeinrichtung 10, die an einer für die Überwachung geeigneten Stelle platziert werden kann und deren Gehäuse 13 hierfür an der Rückseite Haft-Magnete 14 für eine Halterung der Überwachungs- und Anzeigeeinrichtung 10 an Metallteilen aufweist, ist so ausgebildet, daß sie nach dem Einschalten bzw. Aktivieren in einem Bereitschaftszustand oder -modus den Reed-Kontaktes 8 überwacht. Mittels einer ersten Leuchtdiode 15 wird dieser eingeschaltete Zustand bzw. Bereitschaftszustand angezeigt. Eine zweite Leuchtdiode 16 liefert im Alarmfall ein blinkendes Lichtsignal. Weiterhin weist die Überwachungs- und Anzeigeeinrichtung 10 einen akustischen Signalgeber 17 auf, der beispielsweise von einem Piezo-Signalgeber gebildet ist und im Alarmfall ein akustisches Signal in Form einer impulsförmigen Tonfolge abgibt.

Im einzelnen ist die Steuer- und Überwachungseinrichtung 10 so ausgebildet, daß wenn, wenn beim Aktivieren oder im Bereitschaftszustand eine Lockerung oder gar Trennung der Verbindung 1 erfolgt und sich hierbei der Flansch 4 mit dem Permanentmagneten 5 über ein vorgegebenes Maß x von dem Sensorelement 6 wegbewegt hat, der Reed-Kontakt 8 öffnet und hierdurch ein das optische Signal an der Leuchtdiode 16 sowie das akustische Signal am Signalgeber 17 veranlassendes Steuersignal erzeugt wird. Bevorzugt ist die Steuer- und Überwachungseinrichtung 10 so ausgeführt, daß ein einmal ausgelöster optischer und akustischer Alarm erst durch Drücken einer hierfür vorgesehenen Funktionstaste 18 beendet werden kann, und zwar auch dann, wenn die Verbindung 1 wiederum ordnungsgemäß hergestellt ist. Selbstverständlich ist der Abstand x, ab dem der Alarm ausgelöst wird, wesentlich kleiner als die Länge 3', mit der das betreffende Ende des Tubus 3 in das den Anschluß 2''' bildende Ende des Y-Stückes 2 eingeführt ist. Hierdurch ist sichergestellt, daß der Alarm auf jeden Fall vor einem Trennen der Verbindung 1 ausgelöst wird.

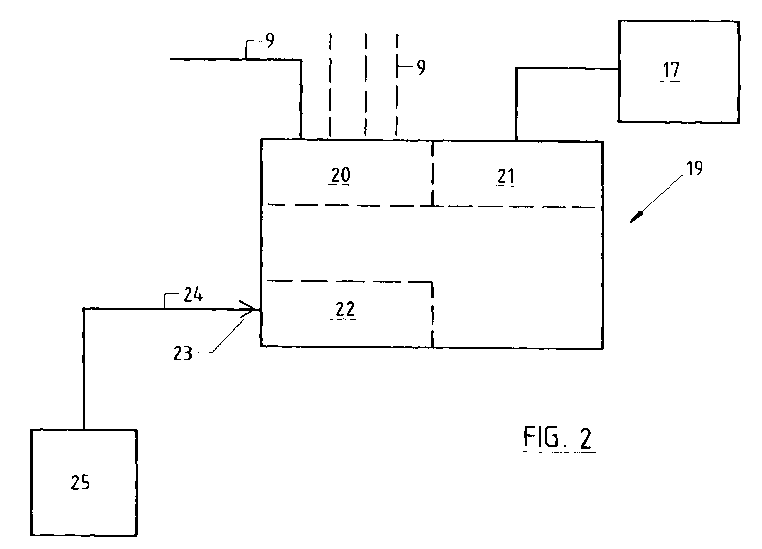

Die Fig. 2 zeigt die Überwachungs- und Anzeigeeinrichtung 10 als Blockdiagramm, und zwar zusammen mit der von dem Permanentmagneten 5 und dem Reed-Kontakt 8 gebildeten Sondeneinrichtung. Mit 19 ist die eigentliche Elektronik bezeichnet, die neben einem Eingangsschaltkreis 20 und einer Ausgangsstufe 21 zum Ansteuern des akustischen Signalgebers die beispielswese mit diskreten Bauteilen oder mit integrierten Schaltkreisen aufgebaute Überwachungselektronik bildet. Diese Überwachungselektronik kann aber auch von einem Mikroprozessor mit zugehörigen Elementen gebildet sein.

Teil der Überwachungs- und Anzeigeeinrichtung 10 ist weiterhin eine im Gehäuse 13 untergebrachte wiederaufladbare Batterie 22, die über einen entsprechenden Anschluß 23 am Gehäuse 13 und ein Ladekabel 24 mit einem Netzgerät 25 verbunden werden kann.

Grundsätzlich kann die die Batterie 22 umfassende Stromversorgung für die Elektronik 19 so ausgeführt sein, daß ein Betrieb der Überwachungs- und Anzeigeeinrichtung 10 auch bei angeschlossenem Netzgerät 25 möglich ist, so daß beim Betrieb auch ein Aufladen der Batterie 22 erfolgt oder bei erschöpfter Batterie 22 durch den Anschluß des Ladegerätes 25 der Betrieb der Überwachungs- und Anzeigeeinrichtung 10 möglich ist.

Bevorzugt ist aber die Stromversorgung für die Elektronik 19 so ausgebildet, daß eine Funktion der Überwachungs- und Anzeigeeinrichtung 10 nur dann möglich ist, wenn das Ladekabel 24 nicht an diese Einrehtung angeschlossen ist. Hierdurch werden Störungen und insbesondere auch Gefährdungen durch ein defektes Ladegerät 25 während des Betriebes des Überwachungssystems vermieden.

Durch den Batteriebetrieb ist die Uberwachungs- und Anzeigeeinrichtung sehr mobil und kann an jeder geeigneten Stelle platziert werden.

Bevorzugt werden mit einer Einrichtung 10 mehrere Verbindungen 1 bzw. die an diesen Verbindungen vorgesehenen und von jeweils einem Permanentmagneten 5 und einem Sensorelement 6 gebildeten Sensoreinrichtungen überwacht. Hierfür weist die Eingangsschaltung 20 mehrere von jeweils einer Steckerbuchse 12 gebildete Eingänge auf, die jeweils über eine Verbindungsleitung 9 mit der Sensoreinrichtung an einer Verbindung 1 verbunden sind. Mit der Eingangsschaltung 20 werden dann diese Eingänge bei aktivierter Überwachungs- und Anzeigeeinrichtung 10 gleichzeitig überwacht oder ständig periodisch abgefragt. Ist eine der Verbindungen 1 nicht ordnungsgemäß und ist daher der dieser Verbindung zugeordnete Reed-Kontakt 8 geöffnet, so erfolgt der optische und akustische Alarm.

Um bei mehreren Eingängen bzw. bei der Überwachung mehrerer Verbindungen 1 die nicht ordnungsgemäße Verbindung schnell auffinden zu können, ist an der Einrichtung 10 bzw. an dem Gehäuse 13 eine optische Anzeige 26 vorgesehen, mit der die defekte Verbindung 1 bzw. der zugehörige Ausgang (Steckerbuchse 12) angezeigt wird. Diese Anzeigeeinrichtung besteht beispielsweise aus einer Vielzahl von Leuchtdioden, von denen jedem Ausgang jeweils eine zugeordnet ist.

Die Überwachungs- und Anzeigeeinrichtung 10 bzw. deren Elektronik 19 ist weiterhin so ausgeführt, daß im Bereitschaftszustand nur ein minimaler Stromverbrauch erfolgt, der sich im wesentlichen aus den über die Reed-Kontakte 8 fließenden Ruheströmen sowie aus dem geringen Strom für die den Betriebszustand anzeigende Leuchtdiode 15 zusammensetzt. Erst im Alarmfall besteht dann insbesondere für die Abgabe des akustischen Signals ein höherer Strombedarf.

Bei der vorstehenden Beschreibung wurde davon ausgegangen, daß das Sensorelement 6 für die jeweils zu überwachende Verbindung 1 über ein Verbindungskabel 19 an die Überwachungs- und Anzeigeeinrichtung 10 angeschlossen ist. Grundsätzlich kann diese Verbindung auch drahtlos erfolgen, und zwar beispielsweise mit Hilfe von elektromagnetischen Wellen oder bevorzugt mit Hilfe von Infrarot-Licht. In diesem Fall ist dann jedes Sensorelement 6 mit einem Mini-Sender für ein entsprechendes elektromagnetisches Signal oder ein Infrarot-Signal und mit eigener Stromversorgung (Batterie) ausgeführt. Die Überwachungs- und Anzeigeeinrichtung 10 besitzt einen Empfänger für Signale in Form von elektromagnetischen Wellen oder Infrarot-Licht. Dieser Empfänger ist dann beispielsweise Teil der Eingangsschaltung 20. Sollen mehreren Verbindungen 1 gleichzeitig überwacht werden, so liefern die den einzelnen Sensorelementen 6 zugeordneten Sender unterschiedliche Signale, die nach einer Demodulation und/oder Decodierung im Empfänger den einzelnen Verbindungen 1 zugeordnet werden. Fehlt das Signal eines Senders, so erfolgt auf jeden Fall der optische und akustische Alarm mittels der Leuchtdiode 16 und des Signalgebers 17.

Um bei mehreren Anschlüssen 12 auf eine der Anzahl dieser Anschlüsse geringere Anzahl von Verbindungen 1 überwachen zu können, sind bevorzugt Blind-Stecker 27 vorgesehen, die anstelle des Steckers 11 in die nicht belegten Anschlüsse bzw. Buchsen 12 eingesteckt werden und einen geschlossenen Reed-Kontakt 8 simulieren. Anstelle dieser Blind-Stecker kann an der Überwachungs- und Anzeigeeinrchtung 10 bzw. am Gehäuse 13 auch ein jedem Eingang bzw. jeder Buchse 12 zugeordneter Schalter 28 vorgesehen sein, der bei nicht belegter Buchse 12 in die geschlossene Stellung geschaltet und dadurch einen geschlossenen Reed-Kontakt simuliert.

Die Erfindung wurde voranstehend an einem Ausführungsbeispiel beschrieben. Es versteht sich, daß Änderungen sowie Abwandlungen möglich sind, ohne daß dadurch der der Erfindung zugrundeliegende Erfindungsgedanke verlassen wird.

So ist es grundsätzlich möglich, anstelle der den Reed-Kontakt 8 aufweisenden Sensoreinrichtung 6 auch andere Sensoren zu verwenden, mit denen berührungslos das Vorhandensein eines am Tubus 3 vorgesehenen Gegenstücks an einem solchen Sensor überwacht werden kann. In diesem Sinne eignen sich insbesondere auch induktive oder kapazitive, berührungslos arbeitende Näherungs-Sensoren, wenngleich das Sensorelement 6 mit dem Reed-Kontakt 8 eine besonders robuste Ausführung darstellt, die insbesondere auch zusätzlich zu den Anschlüssen des Reed-Kontaktes keine weitere Versorgungsanschlüsse usw. benötigt. Anstelle des Reed-Kontaktes 8 ist auch ein auf ein Magnetfeld ansprechender Sensor (Hall-Sensor verwendbar.

Durch die abnehmbare Befestigung des Sensorelemente s 6 von dem Y-Stück 2 kann dieses auch in der erforderlichen Weise gereinigt und sterilisiert werden. Durch die CLIP-Befestigung des Sensorelementes 6 ist eine schnelle und problemlose Herstellung der die Verbindung 1 überwachenden Sensoranordnung möglich.

### Bezugszeichenliste

- 1: Verbindung
- 2: Y-Stück
- 2', 2'', 2''': Anschluß
- 3: Tubus
- 4: Flansch
- 5: Permanentmagnet
- 6: Sensorelement
- 7: Gehäuse
- 8: Reed-Kontakt
- 9: Verbindungskabel
- 10: Überwachungs- und Anzeigeeinrichtung
- 11: Stecker
- 12: Buchse
- 13: Gehäuse
- 14: Haftmagnet
- 15, 16: Leuchtdiode
- 17: Signalgeber
- 18: Funktionstaste
- 19: Elektronik
- 20: Eingangsschaltung
- 21: Ausgangsstufe
- 22: Batteri
- 23: Ladeanschluß
- 24: Ladekabel
- 25: Netzgerät
- 26: Anzeige
- 27: Blindstecker
- 28: Schalter

## Patentansprüche

1. Medizinisches Beatmungssystem, bestehend aus wenigstens zwei Elementen, von denen eines ein Y-Stück und eines ein mit diesem Y-Stück verbundener Tubus (3) ist, **gekennzeichnet** durch ein erstes, im Bereich der Verbindung (1) an einem ersten der beiden Elemente vorgesehenes Sensorelement (4) sowie durch ein zweites, an der Verbindung (1) an dem zweiten der beiden Elemente vorgesehenes Sensorelement (6), von denen wenigstens ein Sensorelement mindestens einen Signalgeber (8) aufweist, der dann, wenn sich die beiden Sensorelemente (4, 6) über einen maximal vorgegebenen Betrag voneinander entfernen, ein einen Alarm bewirkendes Signal veranlaßt, sowie durch eine Überwachungs- und Anzeigeeinrichtung (10), die beim Ansprechen des Signalgebers (8) ein akustisches und/oder optisches Alarmsignal abgibt, wobei das erste Sensorelement (4) wenigstens einen Permanentmagneten (5) aufweist oder bildet, und wobei der Signalgeber des zweiten Sensorelementes (6) von wenigstens einem magnetisch betätigbaren Kontakt, vorzugsweise von einem Reed-Kontakt (8), oder von einem auf ein Magnetfeld ansprechenden elektronischen Sensor, z.B. Hall-Sensor gebildet ist.

2. Einrichtung nach Anspruch 1, dadurch gekennzeichnet, daß die Überwachungs- und Anzeigeeinrichtung (1) eine getrennt von den Sensorelementen (4, 6) anzuordnende Einrichtung ist.

3. Einrichtung nach Anspruch 2, dadurch gekennzeichnet, daß die Überwachungs- und Anzeigeeinrichtung (10) ein Gehäuse (13) mit wenigstens einer, vorzugsweise von wenigstens einem Haftmagneten (14) gebildete Halterung zur Befestigung des Gehäuses (13) aufweist.

4. Einrichtung nach einem der Ansprüche 1 - 3, dadurch gekennzeichnet, daß die Überwachungs- und Anzeigeeinrichtung (10) durch eine Batterie, vorzugsweise durch eine wiederaufladbare Batterie (22) betrieben ist.

5. Einrichtung nach einem der Ansprüche 1 - 4, dadurch gekennzeichnet, daß das erste und/oder zweite Sensorelement (4) fest an dem zugehörigen Element (3) der Verbindung (1) vorgesehen ist, vorzugsweise einstückig mit diesem Element (3) der Verbindung hergestellt ist.

6. Einrichtung nach einem der Ansprüche 1 - 5, dadurch gekennzeichnet, daß wenigstens ein Sensorelement (6) abnehmbar an dem zugehörigen Element (2) der Verbindung (1) vorgesehen ist.

7. Einrichtung nach einem der Ansprüche 1 - 6, dadurch gekennzeichnet, daß das zweite Sensorelement (6) über ein Verbindungskabel (9) oder drahtlos für eine drahtlose Datenübertragung mit einem Eingang (12) der Überwachungs- und Anzeigeeinrichtung (10) verbunden ist.

8. Einrichtung nach einem der Ansprüche 1 - 7, dadurch gekennzeichnet, daß einer Überwachungs- und Anzeigeeinrichtung (10) wenigstens zwei jeweils eine Verbindung (1) überwachende und von wenigstens einem ersten und einem zweiten Sensorelement (4, 6) gebildete Sensoreinrichtungen zugeordnet sind.

9. Einrichtung nach einem der Ansprüche 1 - 8, dadurch gekennzeichnet, daß die Überwachungs- und Anzeigeeinrichtung (10) eine Elektronik (19) aufweist, die nach dem Auslösen eines Alarms diesen auch bei Wiederherstellung der gestörten Verbindung (1) bis zum Betätigen einer Funktions- oder Alarm-Löschtaste (18) der Überwachungs- und Anzeigeeinrichtung aufrecht erhält.

## Claims

1. Medical respiration system, comprising at least two elements, of which one is a Y-piece and one is a tube (3) connected with this Y-piece, characterized by a first sensor element (4) provided in the area of the connection (1) on the first of the two elements, as well as by a second sensor element (6) provided on the connection (1) on the second of the two elements, of which one sensor element has at least one signal transmitter (8) which triggers a signal which effects an alarm when the two sensor elements (4, 6) become distanced from one another above a maximum set amount; as well as by a monitoring and display device (10) which emits an acoustic and/or optical alarm signal when the signal transmitter (8) responds, with the first sensor element (4) having or forming at least one permanent magnet (5), and with the signal transmitter of the second sensor element (6) being formed by at least one magnetically operable contact, preferably by a reed contact (8) or by an electronic sensor, e.g. Hall sensor, which responds to a magnetic field.

2. Device in accordance with claim 1, characterized in that the monitoring and display device (1) is a device which is to be arranged separate from the sensor elements (4, 6).

3. Device in accordance with claim 2, characterized in that the monitoring and display device (10) has a housing (13) with at least one holding device, preferably formed by at least one adhesive magnet (14), for fixing the housing (13).

4. Device in accordance with one of the claims 1-3, characterized in that the monitoring and display device (10) is operated by a battery, preferably by a rechargeable battery (22).

5. Device in accordance with one of the claims 1-4, characterized in that the first and/or second sensor element (4) is provided firmly fixed on the corresponding element (3) of the connection (1), preferably produced in one piece with this element (3) of the connection.

6. Device in accordance with one of the claims 1-5, characterized in that at least one sensor element (6) is provided on the corresponding element (2) of the connection (1), in such a way as to be detachable.

7. Device in accordance with one of the claims 1-6, characterized in that the second sensor element (6) is connected, via a connection cable (9) or by wireless means for a wireless data transmission, with an input (12) of the monitoring and display device (10).

8. Device in accordance with one of the claims 1-7, characterized in that assigned to a monitoring and display device (10) are at least two sensor devices which each monitor one connection (1) and which are formed by at least a first and a second sensor element (4, 6).

9. Device in accordance with one of the claims 1-8, characterized in that the monitoring and display device (10) has electronics (19) which, after triggering an alarm, maintain it even after the disrupted connection (1) has been reinstated, until a function key or alarm reset key (18) on the monitoring and display device has been operated.

## Revendications

1. Système médical de respiration artificielle constitué d'au moins deux éléments, dont l'un est un élément en forme de Y et l'autre un tube (3) raccordé à cet élément en Y, caractérisé par un premier élément de détection (4) disposé dans la zone de la jonction (1) sur un premier des deux éléments, ainsi que par un second élément de détection (6) disposé à la jonction (1) sur le second des deux éléments, au moins un élément de détection présentant au moins un transmetteur de signaux (8), qui, lorsque les deux éléments de détection (4,6) s'écartent l'un de l'autre d'une distance maximale prédéterminée, délivre un signal déclenchant une alarme, ainsi que par un dispositif de surveillance et d'affichage (10), qui délivre, lors de la réaction du transmetteur de signaux (6), un slgnal d'alarme acoustique et/ou optique, le premier élément de détection (4) présentant ou formant au moins un aimant permanent (5), et le transmetteur de signaux du second élément de détection (6) étant formé d'au moins un contact commandé magnétiquement, de préférence d'un contact Reed (6), ou d'un détecteur électronique sensible au champ magnétique, par exemple un détecteur Hall.

2. Dispositif selon la revendication 1, caractérisé en ce que le dispositif de surveillance et d'affichage (1) est un dispositif qui est aménagé séparément des éléments de détection (4, 6).

3. Dispositif selon la revendication 2, caractérisé en ce que de dispositif de surveillance et d'affichage (10) présente un boîtier (13) avec au moins un support, de préférence formé d'au moins un aimant adhérent (14), pour fixer le boîter (13).

4. Dispositif selon l'une quelconque des revendications 1 à 3, caractérisé en ce que le dispositif de surveillance et d'affichage (10) est commandé par une batterie, de préférence par une batterie rechargeable (22).

5. Dispositif selon l'une quelconque des revendications 1 à 4, caractérisé en ce que le premier et/ou le second élément de détection (4) sont fixés à l'élément correspondant (3) de la jonction (1), de préférence fabriqués d'une seule pièce avec cet élément (3) de la jonction.

6. Dispositif selon l'une quelconque des revendications 1 à 5, caractérisé en ce qu'il est prévu au moins un élément de détection amovible (6) sur l'élément correspondant (2) de la jonction (1).

7. Dispositif selon l'une quelconque des revendications 1 à 6, caractérisé en ce que le second élément de détection (6) est raccordé à une entrée (12) du dispositif de surveillance et d'affichage (10) par l'entremise d'un câble de raccordement (9) ou sans fil pour une transmission de données sans fil.

8. Dispositif selon l'une quelconque des revendications 1 à 7, caractérisé en ce qu'au moins deux dispositifs de détection surveillant respectivement une jonction (1) et formés d'au moins un premier et un second éléments de détection (4, 6) sont affectés à un dispositif de surveillance et d'affichage (10).

9. Dispositif selon l'une quelconque des revendications 1 à 8, caractérisé en ce que le dispositif de surveillance et d'affichage (10) présente un circuit électronique (19), qui, après le déclenchement d'une alarme, maintient celle-ci, même lors de la restauration de la jonction déréglée (1), jusqu'à l'actionnement d'une touche de fonction ou de suppression d'alarme (18) du dispositif de surveillance et d'affichage.
